# EUROPÄISCHE PATENTSCHRIFT

(11) **EP 1 729 745 B1**
(45) Veröffentlichungstag und Bekanntmachung des Hinweises auf die Patenterteilung: **20.06.2012**
(21) Anmeldenummer: 05716128.3
(22) Anmeldetag: 16.03.2005
(51) Int. Cl.: A61K 9/50, B01J 13/02

(54) **Verfahren zur Herstellung von CS-Partikeln und Mikrokapseln unter Verwendung poröser Template, CS-Partikel und Mikrokapseln sowie deren Verwendung**
Method for producing cs particles and microcapsules using porous templates, cs particles and microcapsules, and the use thereof
Procede pour produire des particules de type coeur-ecorce et des microcapsules au moyen de matrices microporeuses, particules de type coeur-ecorce et microcapsules, et leur utilisation

(30) Priorität: 19.03.2004 DE 102004013637
(43) Veröffentlichungstag der Anmeldung: 13.12.2006
(73) Patentinhaber: Capsulution Pharma AG, 12489 Berlin (DE)
(72) Erfinder: DÄHNE, Lars, 12587 Berlin (DE); BAUDE, Barbara, 14548 Schwielowsee (DE)
(74) Vertreter: Vossius & Partner
(86) Internationale Anmeldenummer: PCT/EP2005/002810
(87) Internationale Veröffentlichungsnummer: WO 2005/089727

(56) Entgegenhaltungen:
- WO-A-01/51196
- WO-A-99/47252
- WO-A-03/090920
- US-A1- 2002 172 716
- US-B1- 6 479 146
- WANG, D. AND CARUSO, F.: "Polyelectrolyte-coated colloid spheres as templates for sol-gel reactions" CHEM. MATER., Bd. 14, 2002, Seiten 1909-1913, XP002339641

## Beschreibung

Die Erfindung liegt auf dem Gebiet der Kolloidtechnik und betrifft ein Verfahren zur Herstellung von CS-(core-shell) Partikel und gefüllten Mikrokapseln unter Verwendung poröser Template sowie CS-Partikel und Mikrokapseln.

Mikrokapseln aus alternierend adsorbierten Polyelektrolytschichten (Layer by Layer, LbL) sind beispielsweise aus [1] bekannt und in DE 198 12 083 A1, DE 199 07 552 A1, EP 0 972 563 A1, WO 99/47252 und US 6,479,146 beschrieben, deren Offenbarungsinhalt hiermit vollständig aufgenommen wird. Derartige Kapselsysteme besitzen aufgrund ihrer einstellbaren Sernipermeabilität ein hohes Anwendungspotential als Mikroreaktoren, Drug Delivery Systeme etc. Voraussetzung ist die Befüllung mit entsprechenden Wirkstoffen, Enzymen, Polymeren oder Katalysatoren. Weitere Kapselsysteme sind aus WO 01/51196 und WO 03/090920 bekannt:

Die internationale Anmeldung WO 01/51196 offenbart einen festen Partikelkern, der mit einer amphiphilischen Substanz umhüllt wird und danach mit Polyelektrolyten oder Nanopartikeln gecoated wird. Der Kern besteht aus Wirkstoffen aus den Bereichen Sensorik, Diagnostik, Pharmazie, Medizin, Nahrungsmittelindustrie, Biotechnologie, Kosmetik. Die Wirkstoffen können auch nach dem Auflösen des porösen Kerns mittels Lösungsmittel in die leere Kapselhülle eingeführt werden. Die Porosität des Produkts wird bestimmt durch die amphiphilische Hülle. Die Hülle besteht aus Polyelektrolyten. Die amphiphilische Hülle ist notwendig um die weiteren alternierend geladenen Schichten von Polyelektrolyten aufzutragen. Sie wird als Grundierungsschicht angesehen. Die Mikrokapseln sind 50 nm bis 10µm groß.

Mikropartikel in der WO99/47252 sind für Wirkstoffe durchlässig und die fertigen Kapseln sind 1-50µm groß. Die Templatpartikel werden mit entgegengesetzt geladenen Polyelektrolyten umhüllt. Die chemische Struktur der Polyelektrolyten bestimmt die Wandporosität. Auf eventuell ungeladene Kerne wird ein geladener Precursorfilm aufgetragen. Zum Schluss wird der poröse Kern des umhüllten Mikropartikels aufgelöst, so dass eine leere Kapsel bleibt. Der Wirkstoff wird an das Templatpartikel (Kern) gekoppelt und wird ausgewählt aus Katalysatoren, Enzymen, pharmazeutischen Wirkstoffen, Sensormolekülen, Polymeren, Farbstoffen, Pflanzenschutzmitteln, Aromastoffen und Gasen. Die Permeabilität der Polyelektrolythülle kann weiter bestimmt werden durch Aufbringen einer Lipidschicht.

Weiterhin sind Trennmembranen aus stoffdurchlässigen und mit Polyelektrolytschichten beschichteten Verbundwerkstoffen aus DE 100 31 281 A1 bekannt.

Bisher wurden überwiegend LbL-Mikrokapseln hergestellt, die im Inneren das gleiche Medium (Lösungsmittel) wie außerhalb aufweisen. Bei den meisten Anwendungen werden jedoch im Inneren funktionalisierte Makromoleküle benötigt, die dort permanent immobilisiert und in gelöster Form vorliegen sollen, um ihre Funktionalität zu erhalten. Die bekannten Verfahren zur Herstellung derartig befüllter Kapseln sind nur unter bestimmten Bedingungen einsetzbar. Speziell für empfindliche Biomoleküle treten Schwierigkeiten beim Befüllen nach den bekannten Methoden auf.

Bisher sind 4 Möglichkeiten zur Befüllung mit Makromolekülen entwickelt worden.
a) Ship in bottle synthesis^{[2]}
   Monomere werden in Gegenwart von Kapseln polymerisiert. Die kleinen Initiator- und Monomermoleküle dringen in die Kapseln ein. Nach der Polymerisation werden die in der Lösung synthetisierten Polymere weggewaschen, wogegen die im Inneren der Kapseln entstandenen Polymere nicht mehr durch die Kapselwand diffundieren können und im Inneren immobilisiert sind. Diese Methode ist allerdings auf synthetische Moleküle beschränkt.
b) Switching of the permeability^{[3.4]}
   Hierbei werden Kapseln verwendet, deren Permeabilität sich bei Änderung des pH-Werts oder der Ionenstärke der Lösung verändert. Die Kapseln werden zu einer Lösung des Makromoleküls gegeben und durch Zugabe von Salz oder Änderung des pH-Werts in einen durchlässigen Zustand geschaltet. Nach dem Eindringen der Polymere wird der pH-Wert wieder zurückgeschaltet bzw. das Salz weggewaschen und die Makromoleküle sind im Inneren immobilisiert. Der Nachteil liegt in der Notwendigkeit, spezielle, schaltbare Kapseln zu verwenden. Weiterhin können bisher nur geringe Konzentrationen verkapselt werden.
c) Controlled precipitation^{[5]}
   Bei dieser Methode wird die Füllung vor dem Aufbringen der LbL Schale auf dem Templat präzipitiert. Dazu wird entweder eine geringe Löslichkeit in einem spezifischen Lösungsmittel oder eine Komplexierung des Makromoleküls mit einem Hilfsstoff ausgenutzt. Auf die Präzipitatschicht wird anschließend eine normale LbL Beschichtung aufgebracht. Das Templat wird aufgelöst. Durch Veränderung des Lösungsmittels oder die Zersetzung des Komplexes wird das Makromolekül von der inneren Oberfläche der Kapseln in das Kapselinnere gelöst. Diese Methode muss für jedes Füllmaterial sehr spezifisch optimiert werden, was bisher nur an ausgewählten Beispielen gelungen ist. Häufig sind die Präzipitatschichten nicht homogen genug (raue Oberfläche) um eine gut definierte LbL Kapsel aufzubringen. Weiterhin muss im Falle von Biopolymeren die Auflösung des Templates unter milden Bedingungen erfolgen, was bei den meist verwendeten Melamin-Formaldehyd-(pH 1), Erythrozyt- (pH 12, NaOCl) und Polystyrolpartikeln (Tetrahydrofuran) nicht der Fall ist.
d) Porous CaCO₃ templates^{[9]}
   Bei diesem Ansatz werden poröse CaCO₃ Template verwendet. Die Template werden in Lösungen von abwechselnd geladenen Polyelektrolyten suspendiert. Dabei werden die äußere und die innere Oberfläche der porösen Template mit Polyelektrolyten beschichtet. Nach Auflösen des CaCO₃ Templats mit EDTA verbleiben Mikrokapseln mit einem inneren Gerüst aus Polyelektrolyten, das von einer nicht geschlossenen Polyelektrolythülle umgeben ist. An das innere Gerüst der Mikrokapseln lassen sich dann nachfolgend Makromoleküle anlagern. Mit diesem Verfahren lassen sich keine geschlossenen Kapselhülle herstellen, da die Poren der CaCO₃ Template relativ groß sind.

Aufgabe der vorliegenden Erfindung ist es daher, ein Verfahren zum Verkapseln von Materialien bzw. Wirkstoffen anzugeben, bei dem die zu verkapselnden Wirkstoffe einfach und in hoher Konzentration im Inneren der Kapseln angereichert werden können.

Erfindungsgemäß wird diese Aufgabe durch ein Verfahren zur Herstellung von CS-Partikeln und/oder Mikrokapseln mit den Schritten gelöst:
- poröse Templates werden bereitgestellt, wobei es sich bei den Templaten um poröse organische und/oder anorganische Mikropartikel mit einem Durchmesser kleiner als 100 um handelt
- zumindest ein zu verkapselnder Wirkstoff wird in den porösen Templaten adsorbiert
- zumindest eine Grundierungsschicht wird auf die porösen Template aufgebracht und - eine Kapselhülle wird um die mit der Grundierungsschicht versehenen porösen Template durch Aufbringen von alternierend geladenen Polyelektrolyt-und/oder Nanopartikelschichten auf die porösen Template gebildet
- wobei die Grundierungsschicht aus einem Material gebildet wird, das die Poren der porösen Template verschließt und für die zur Herstellung der Kapselhülle verwendeten Beschichtungsmaterialien weitgehend undurchlässig ist.

Dadurch entstehen zunächst CS-Partikel, die noch das poröse Templat als Kern mit dem adsorbierten Wirkstoff enthalten. Nachfolgend kann das poröse Templat aus den CS-Partikeln herausgelöst werden, wobei Mikrokapseln entstehen, die mit dem Wirkstoff befüllt sind. Vor Bildung der Kapselhüllen kann zumindest eine Grundierungsschicht auf die porösen Template aufgebracht werden. Ggf. werden noch zusätzliche Schichten aus Polyelektrolyten und/oder Nanopartikeln vor der Bildung der eigentlichen Kapselhülle auf die Grundierungsschicht aufgebracht. Typischerweise werden die Kapselhüllen durch sequentielles Adsorbieren alternierend geladener Polyelektrolyte hergestellt (sog. LbL-Verfahren). Typischerweise werden viele als kolloidale Lösung bereitgestellte Template gleichzeitig beschichtet, so dass jedes Templat mit einer Kapselhülle versehen wird. Im Ergebnis wird dann eine kolloidale Lösung von CS-Partikeln bzw. nach Auflösen der Template eine kolloidale Lösung von Mikrokapseln erhalten, die ggf. weiterbehandelt werden können.

Im Rahmen der vorliegenden Erfindung werden unter porösen Templaten solche Teilchen verstanden, die eine Vielzahl von Poren bzw. inneren Hohlräumen aufweisen. Nach dem Aufbringen der LbL Schichten und ggf. einer Grundierungsschicht auf die porösen Template werden Kern-Schale-(Core-Shell, CS)-Teilchen erhalten, welche als CS-Partikel bezeichnet werden. Nach dem Herauslösen des ursprünglichen porösen Templates existiert nur noch die Schale, d.h. die Kapselhülle ggf. mit innerer Grundierungsschicht, die im einfachsten Fall mit dem äußeren Lösungsmittel oder mit einer Lösung oder Suspension des eingeschlossenen Wirkstoffes gefüllt ist. Diese gefüllten Schalen werden als Kapseln bzw. Mikrokapseln bezeichnet. Die CS-Partikel bzw. Mikrokapseln sind mit dem Wirkstoff gefüllt, d.h. der Wirkstoff verbleibt in den CS-Partikeln bzw. Mikrokapseln, da die Kapselhülle als Diffusionsbarriere bezüglich des Wirkstoffs wirkt. Kolloidale Teilchen, die zur Befüllung der porösen Partikel oder zum Aufbau der LbL-Schale verwendet werden und daher im allgemeinen kleiner als 100 nm sind, werden als Nanopartikel bezeichnet.

Im Gegensatz zu den bekannten Methoden bietet die hier beschriebene Erfindung eine neue, einfache und generelle Methode zur Verkapselung von Materialien auch in hoher Konzentration in CS-Partikeln und Mikrokapseln (Layer by Layer Polyelektrolytkapseln). Mit Wirkstoffen befüllte LbL CS-Partikel und Mikrokapseln werden dabei mit Hilfe von porösen Templaten hergestellt. Dazu werden die porösen Template vor der LbL Beschichtung mit einem oder mehreren Wirkstoffen befüllt. Wird der Wirkstoff nur ungenügend in den Poren adsorbiert, können spezielle Hilfsstoffe (Mediatoren) oder pH Änderungen zur Verbesserung der Befüllung genutzt werden. Die befüllten Template werden mit einer speziellen Grundierung überzogen, die nicht in die Poren eindringt, sie jedoch für nachfolgende Beschichtungen abdichtet. Danach erfolgt der Aufbau der Kapselhülle oder -wand über alternierende Adsorption von Polykationen und Polyanionen, wodurch ein gefülltes CS-Partikel entsteht. Für die Herstellung von Mikrokapseln können die porösen Template mit Lösungsmitteln entfernt werden. Speziell bei Silicapartikeln (SiO₂) kann dies unter milden Bedingungen oberhalb von pH 4 durchgeführt werden, um z.B. biologische Wirkstoffe zu schonen

Bei den verwendeten Templaten handelt es sich um poröse Mikropartikel, deren Größe bevorzugt kleiner als 100 µm ist. Die Mikropartikel weisen Poren mit beispielsweise einer Porenweite von 0,3 nm - 100 nm, bevorzugt von 1 nm - 30 nm und besonders bevorzugt von 6 nm - 10 nm auf. Bei vielen Anwendungsfällen kann die untere Grenze der Porenweite zwischen 1 nm und 6 nm, beispielsweise bei 2 nm oder 4 nm, und die obere Grenze der Porenweite zwischen 10 nm und 40 nm, beispielsweise bei 15 nm oder 30 nm, liegen. Grundsätzlich sollte die Porenweite so groß sein, dass die zu verkapselnden Wirkstoffe in die Poren eindringen und sich in den Poren ablagern können, d.h. insbesondere im Inneren der Poren adsorbieren. Bevorzugt sind daher poröse Template mit einer großen inneren Oberfläche, wobei die innere Oberfläche von den Innenwänden der Poren gebildet wird. Insbesondere sollte die effektiv für die Adsorption der Wirkstoffe zur Verfügung stehende innere Oberfläche groß sein. Unter effektiver innerer Oberfläche wird hier der Teil der Oberfläche verstanden, der für die Adsorption eines Wirkstoffs bestimmter Größe tatsächlich zur Verfügung steht. Da die Template häufig Poren mit verschiedener Weite aufweisen, können großmolekulare Wirkstoffe nur in entsprechend große Poren eindringen während für kleinere Moleküle auch die kleineren Poren zur Verfügung stehen. Daher kann die Porengröße auch über die Größe der Nanopartikel oder Moleküle bzw. deren Molekulargewicht beschrieben werden, die noch in die Poren eindringen können. Die untere Grenze des Molekulargewichts liegt bevorzugt bei 100 g/mol Die obere Grenze entspricht einem Molekulargewicht von etwa 5 x 10⁶ g/mol. Dabei spielt auch die Form des einzudringenden Moleküls (gestreckt oder geknäuelt) eine wesentliche Rolle.

Weiterhin ist es möglich, dass die Oberfläche der Porenhohlräume durch mehrere Schichten von alternierend geladenen Polyelektrolyten und/oder Nanopartikeln bevorzugt mit der LbL-Technik beschichtet wird, d.h. es werden Polyelektrolyt-und/oder Nanopartikelschichten an der "inneren" Oberfläche der porösen Template gebildet. Die Größe der Nanopartikel bzw. das Molekulargewicht der Polyelektrolyte ist an die Porenweite entsprechend angepasst. Nach der Auflösung der Template wird ein filigraner Negativabdruck der ursprünglichen Porenstruktur aus unlöslichen Komplexen aus Polyelektrolytkomplexen und/oder Polyelektrolyt /Nanopartikelkomplexen (inneres Gerüst) erhalten, der die Kapseln mechanisch stabilisiert und ihre innere Oberfläche stark erhöht. Der zu verkapselnde Wirkstoff ist in diesem Fall das Material des inneren Gerüsts. Zusätzlich kann vor der Beschichtung oder nach der Auflösung des porösen Templates ein weiterer Wirkstoff in eingelagert und/oder am inneren Gerüst angelagert (z.B. durch Präzipitation und/oder Adsorption) werden, der dann z.B. an das innere Gerüst gebunden ist. Vorteil eines inneren Gerüsts ist neben der mechanischen Stabilisierung eine deutliche Erhöhung der inneren Oberfläche der Mikrokapseln.

Weiterhin wird die Aufgabe durch CS-Partikel gelöst, mit
einem Durchmesser kleiner als 100 µm charakterisiert durch
- einen porösen Kern, in dem zumindest ein Wirkstoff adsorbiert ist,
- einer Grundierungsschicht, die den porösen Kern umgibt und
- einer Kapselhülle aus mehreren Schichten alternierend geladener Polyelektrolyt- und/oder Nanopartikelschichten, wobei die Grundierungsschicht aus einem Material besteht, das die Poren des porösen Kerns verschließt und für die Beschichtungsmaterialen,aus denen die Kapselhülle besteht, weitgehend undurchlässig ist.

Weiterhin wird die Aufgabe gelöst durch Mikrokapseln mit einem Durchmesser kleiner als 100 um
- einer Kapselhülle aus mehreren Schichten alternierend geladener Polyelektrolyt- und/oder Nanopartikelschichten
- einer Grundierungsschicht an der Innenseite der Kapselhülle und
- einem inneren Gerüst aus Polyelektrolytkomplexen und/oder Polyelektrolyt / Nanopartikelkomplexen, das von der Grundierungsschicht und der Kapselhülle umgeben ist.

Es liegt im Rahmen der Erfindung Mikrokapseln mit den folgenden Schritten herzustellen:
- zumindest ein poröses Template wird bereitgestellt, wobei es sich bei dem Template um ein poröses organisches und/oder anorganisches Mikropartikel mit einem Durchmesser kleiner als 100 um handelt
- die Oberfläche der Porenhohlräume des porösen Templates wird mit mehreren Schichten von alternierend geladenen Polyelektrolyten und/oder Nanopartikeln beschichtet
- zumindest eine Grundierungsschicht wird auf das poröse Template aufgebracht
- eine Kapselhülle wird um das mit der Grundierungsschicht versehene poröse Template durch Aufbringen von alternierend geladenen Polyelektrolyt- und/oder Nanopartikelschichten auf das poröse Template gebildet, wobei die Grundierungsschicht aus einem Material gebildet wird, das die Poren des porösen Templates verschließt und für die zur Herstellung der Kapselhülle verwendeten

Beschichtungsmaterialien weitgehend undurchlässig ist und das poröse Templat wird aufgelöst.

Die Polyelektrolyt- und/oder Nanopartikelschichten können ggf. vor oder nach dem Auflösen des Templates noch vernetzt werden (z.B. kovalent), um die Stabilität des Mikrotemplates zu erhöhen. Im Ergebnis liegt wieder ein filigranes Gerüst vor, dass weitgehend einem negativen Abdruck der inneren Porenstruktur des Templates entspricht und hier das Mikrotemplat darstellt. Bei der Beschichtung der Porenoberfläche kann es natürlich auch vorkommen, dass an der Außenseite des Templates Polyelektrolyt- und/oder Nanopartikelschichten gebildet werden, die auch nach dem Auflösen des Templates verbleiben. Je nach Größe der verwendeten Polyelektrolyte und/oder Nanopartikel ist die so gebildete Hülle nur teilweise oder auch weitgehend vollständig aufgebaut. Die gebildeten Mikrotemplate können nun Ausgangspunkt für die Herstellung weiterer Mikropartikel sein, z.B. können Wirkstoffe am Gerüst angelagert werden. Die Mikrotemplate zeichnen sich durch eine relative große Oberfläche bei kleinem Volumen aus und bieten daher viele Bindungsstellen für anzulagernde Wirkstoffe. Im Vergleich zu den Mikrokapseln mit innerem Gerüst wird bei den Mikrotemplaten nach dem Befüllen des Templates mit Polyelektrolyt- und/oder Nanopartikelschichten keine Kapselhülle mit optionaler Grundierungsschicht gebildet. Die Herstellung der Mikrotemplate und der Mikrokapseln mit innerem Gerüst kann jedoch mit gleichen Materialien und unter gleichen Bedingungen erfolgen. Die Größe der Mikrotemplate entspricht der Größe der verwendeten Template und liegt daher in dem weiter oben angegebenen Bereich.

Die hergestellten und mit dem Wirkstoff befüllten CS-Partikel und/oder Mikrokapseln können in vielen Bereichen vorteilhaft angewendet werden, beispielsweise
- zur Verkapselung von Stoffen in den Bereichen der Diagnostik, Sensorik; und/oder
- zur selektiven Akkumulation von Stoffen aus Lösungen für Anwendungen in der Wasserreinigung, Diagnostik, Nuklearchemie etc.; und/oder
- zum Einschluss katalytisch wirkender Stoffe insbesondere Metalle und/oder Metalloxide und/oder Enzyme zur Katalyse chemischer und biochemischer Reaktionen; und/oder
- zur Verkapselung von Nanopartikeln, insbesondere zur Herstellung fluoreszenter oder magnetischer Mikrokapseln, für diagnostische oder medizinische Anwendungen; und/oder
- zur Verkapselung und Freisetzung von Wirkstoffen in der pharmazeutischen und kosmetischen Industrie; und/oder
- zu Separationszwecken, z.B. in der Chromatographie; und/oder
- zu Anwendungen in der Nahrungsmittelindustrie und der Land- und Forstwirtschaft.

Weitere vorteilhafte Ausführungsformen der Erfindung, unabhängig davon, ob es sich um das Verfahren, die CS-Partikel oder Mikrokapseln handelt, werden nachfolgend anhand der Figuren beschrieben. Dabei zeigen:

| | |
|---|---|
| Figur 1 | einzelne Verfahrensschritte des erfindungsgemäßen Verfahrens sowie die dabei erhaltenen CS-Partikel bzw. Mikrokapseln; |
| Figur 2 | a) und b) CS-Partikel und c) Mikrokapseln mit einem verkapselten positiven Polymer; |
| Figur 3 | a) CS-Partikel und b) Mikrokapseln mit einem verkapselten negativen Polymer; |
| Figur 4 | a) CS-Partikel und b) Mikrokapseln mit einem verkapselten zwitterionischen Protein; |
| Figur 5 | a und b) CS-Partikel sowie c und d) Mikrokapseln mit mehreren verkapselten Wirkstoffen; |
| Figur 6 | a) Kapseln mit Nanopartikeln gefüllt b) Superparamagnetismus in mit Nanopartikeln befüllten Kapseln; |
| Figur 7 | a) und b) CS-Partikel sowie c) und d) Mikrokapseln mit einem PAH-Rho/PSS Komplexgerüst gefüllt; und |
| Figur 8 | Verfahrensschritte zur Herstellung von Mikrotemplaten. |

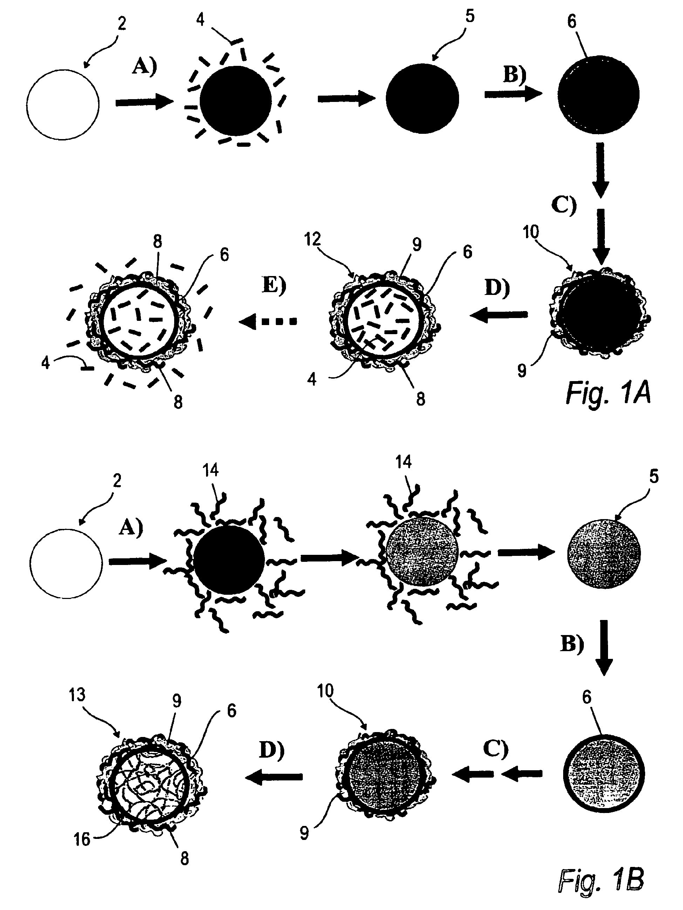

Die einzelnen Verfahrensschritte werden anhand der Figur 1 erläutert. Bevorzugt werden kolloidale Partikel (Template) mit einer definierten Porosität verwendet, die mit den zu verkapselnden Materialien (im folgenden Wirkstoff genannt) in der gewünschten Konzentration befüllt werden können. Figur 1A zeigt das Befüllen mit einem Wirkstoff, der zu einem späteren Zeitpunkt permanent im Inneren immobilisiert ist oder bei entsprechender Wandpermeabilität dosiert freigesetzt wird. Dagegen ist in Figur 1B die Bildung einer Mikrokapsel mit innerem Gerüst dargestellt.

Der Wirkstoff kann jedes Material sein, welches sich
1. im Inneren von porösen Templaten akkumulieren lässt und
2. mit einer LbL Hülle über einen bestimmten Zeitraum zurückhalten lässt.

Die Wirkstoffe können molekular, aggregiert, als Komplex oder in kolloidaler Form vorliegen. Insbesondere handelt es sich bei den zu verkapselnden Wirkstoffen um Polymere und/oder Proteine und/oder organische Moleküle mit Molekulargewichten über 100 g/mol und/oder Nanopartikel. Insbesondere kann es sich dabei um Enzyme und/oder Katalysatoren und/oder Farbstoffe und/oder pharmazeutische bzw. kosmetische Wirkstoffe und/oder Pflanzenschutzmittel handeln. Die zu verkapselnden Wirkstoffe können eine unterschiedliche Affinität bzw. Bindungskonstante hinsichtlich der Ablagerung in den Poren aufweisen. Die Wirkstoffe besetzen die zur Verfügung stehenden Bindungsstellen an der inneren Oberfläche in Abhängigkeit von ihren Bindungskonstanten. Diese unterschiedliche Affinität kann beim Beladen der Template mit mehreren Wirkstoffen ausgenutzt werden.

Poröse Template 2 sind kolloidale anorganische und organische Partikel im für LbL Kapseln geeigneten Größenbereich zwischen 100 nm und 100 µm insbesondere zwischen 500 nm und 15 µm bzw. 30 µm. Bevorzugt ist dabei eine möglichst geringe Verteilung der Porengröße dieser Template 2, d.h. die Poren sollten bevorzugt weitgehend die gleiche Porenweite aufweisen. Insbesondere poröse kolloidale Silicapartikeln und/oder Zeolithe und/oder organische Polymerpartikel eignen sich als Template, da diese Partikel mit einer hinreichend schmalen Verteilung der Porenweite herstellbar sind. Poröse Zeolithpartikel weisen dabei eine Porenweite von insbesondere 0,3 nm bis 10 nm auf.

### Befüllung der Template (Schritt A)

Die Befüllung der porösen Template 2 mit einem oder mehreren Wirkstoffen 4 kann durch attraktive Wechselwirkung vermittelt werden, beispielsweise durch Adsorption der in einer Lösung (beispielsweise ein wässriges Milieu) vorliegenden Wirkstoffe und Template 2 über elektrostatische und/oder H-Brückenbindungen und/oder spezifische Wechselwirkungen und/oder van der Waals Wechselwirkungen erfolgen. Als Wirkstoffe können organische oder anorganische Materialien dienen, für die Layer by Layer Filme impermeabel oder wenig permeabel sind. Diese Materialien können gelöst, als Feststoffgerüst oder in kolloidaler Form als Nanopartikel vorliegen. Für die Adsorption in den porösen Templaten besonders geeignet sind elektrostatische Wechselwirkungen, da geladene Materialien zugleich eine gute äußere Oberfläche für die nachfolgenden LbL Beschichtungsschritte bilden. Während die Befüllung mit Molekülen, die entgegengesetzt zur Poren- bzw. Templatoberfläche geladen sind, keine Schwierigkeiten bereitet, erfordern Materialien mit gleichsinniger Ladung eine besondere Behandlung. Dafür kommen insbesondere die drei nachfolgend genannten Varianten in Frage:
1. Durch die Verschiebung des pH-Wertes werden die Wirkstoffe selbst oder die Oberfläche des Templates umgeladen. Dadurch kommt es zu einer attraktiven Wechselwirkung. Insbesondere Biopolymere mit einem isoelektrischen Punkt können so relativ einfach in den Templaten adsorbiert werden.
2. Mittels geeigneter Hilfsstoffe (Mediatoren) wird die Adsorption der Wirkstoffe vermittelt. Die Hilfsstoffe können beispielsweise die Oberfläche der Poren umladen oder auch spezifische Wechselwirkungen zwischen den Wirkstoffen (z.B. Proteine, Peptide, weitere Wirkstoffe) und der Porenoberfläche ermöglichen. Die Verwendung von Hilfsstoffen erlaubt insbesondere eine Umladung von Oberflächen mit nachfolgender Adsorption der gleichsinnig geladenen Wirkstoffe. Derartige Hilfsstoffe können mehrfach, insbesondere 2-5 fach geladene Materialien sein (z.B. Metall³⁺ oder Polyamine (NR₄⁺)ₙ mit n = 2-5 oder SO₄²⁻, PO₄³⁻, R-(COO⁻)ₙ mit n = 2-5) die unter bestimmten Bedingungen (d.h. bei bestimmten pH-Werten und/oder Ionenstärken) einen Komplex mit dem zu adsorbierenden Material bilden und unter anderen Bedingungen (d.h. anderen pH-Werten und/oder Ionenstärken) wieder entfernt werden können.^{[5]}
3. Hilfsstoffe können auch Makromoleküle 14 (Figur 1B) mit einer Ladungszahl n>5 sein, die als gelöste Moleküle oder feste Gerüststrukturen in den Mikrokapseln verbleiben, nachdem die Kerne herausgelöst worden sind. Für die Präparation fester Gerüststrukturen werden LbL Strukturen in den Poren durch abwechselnde Adsorption vergleichsweise niedermolekularer Polyelektrolyte erzeugt. Die gebildeten Strukturen entsprechen einem Negativabdruck der vorhandenen Poren und lassen sich im Allgemeinen nachfolgend nicht mehr auflösen. Nach dem Herauslösen des ursprünglichen Templates bleiben sie als filigranes Geflecht oder inneres Gerüst 16 (Figur 1 B) mit sehr großer Oberfläche im Inneren der Mikrokapseln zurück. Derartige Strukturen können zur mechanischen Stabilisierung von Mikrokapseln verwendet werden oder durch die große Oberfläche zur Akkumulation anderer Materialien oder für Katalysatoranwendungen dienen.

Sollen mehrere Wirkstoffe verkapselt werden, kann deren Adsorption nacheinander oder gleichzeitig erfolgen. Zur Befüllung mit den Wirkstoffen werden Porengrößen verwendet, die auf die Größe der zu befüllenden Moleküle abgestimmt sind. Insbesondere bei Silicapartikeln lassen sich Moleküle zwischen 0,1 und 5000 kDa (100 g/mol - 5 000 000 g/mol) in Porengrößen von 4 bis 30 nm einlagern. Es können auch mehrere Wirkstoffe bei vergleichbaren Bindungskonstanten simultan oder bei unterschiedlichen Bindungskonstanten sequentiell eingelagert werden. Dabei wird der Wirkstoff mit der höheren Bindungskonstante im Unterschuß befüllt, d.h. dessen Konzentration wird so gewählt, dass dieser Wirkstoff nicht alle zur Verfügung stehenden Bindungsstellen besetzt. Danach werden die unvollständig gefüllten Partikel mit dem 2. Wirkstoff in einer Lösung durch Adsorption aufgefüllt. Im Ergebnis sind die Template 4 weitgehend mit dem oder den Wirkstoff(en) 4 aufgefüllt.

### Grundierung (Schritt B)

Auf die nun gefüllten Template 5 wird optional eine Grundierungsschicht 6 aus beispielsweise einem Polyelektrolyt oder aus Nanopartikeln aufgebracht. Das Grundierungsmaterial ist so auszuwählen und anzupassen, dass es
1. selbst nicht in die gefüllten Poren eindringt,
2. ein Eindringen der beim nachfolgenden Aufbau der LbL Kapselhülle verwendeten Materialien ins Innere verhindert.
   Besonders geeignet sind hochmolekulare oder/und verzweigte Polyelektrolyte sowie Nanopartikel, die an die Größe der Poren angepasst sind. Das Grundierungsmaterial 6 unterscheidet sich typischerweise von den nachfolgend aufzubringenden Materialien der Hülle. Ggf. kann es sich auch um einen Polyelektrolyt handeln, der jedoch ein höheres Molekulargewicht und/oder eine verzweigtere Struktur und/oder eine bessere Vernetzung als die LbL Kapselhüllmaterialien aufweist. Dafür kann ein extra Vernetzungsschritt z.B. über Glutaraldehyd bei aminofunktionalisierten Polyelektrolyten durchgeführt werden.

### Beschichtung (Schritt C)

Anschließend werden auf diese Grundierungsschicht 6 alternierende Schichten 8 aus kationisch und anionisch geladenen Stoffen (Polyelektrolyte), bevorzugt Polymere, aufgebracht, bis die gewünschte Semi- oder Impermeabilität der LbL Kapselwand 9 für den eingeschlossenen Stoff erreicht ist. Die Permeabilität der LbL Kapsel kann dabei durch die Schichtzahl, die Auswahl des Materials, durch eine Nachbehandlung mittels Annealing, oder durch Implementierung von weiteren Stoffen in die Kapselwand^{[8]} für das jeweilige verkapselte Material speziell eingestellt werden. Nach dem Aufbau der Kapselwand liegen CS-Partikel 10 mit einem gefüllten porösen Kern vor. Geeignete Stoffe zur Bildung der Kapselwand sowie geeignete Verfahrensabläufe lassen sich den bereits genannten Dokumenten DE 198 12 083 A1, DE 199 07 552 A1, EP 0 972 563 A1, WO 99/47252 und US 6,479,146 entnehmen.

### Auflösen der Kerne (Schritt D)

Eine nachfolgende optionale Auflösung der Kerne (Template) aus den CS-Partikeln 10 erfolgt mit einem geeigneten Lösungsmittel. Die Produkte der Auflösung werden durch Waschen mit dem Lösungsmittel und Wasser aus dem Kapselinneren entfernt, wobei der eingefüllte Wirkstoff 4 mit größerem Molekulargewicht im Inneren zurückbleibt. Lösungsmittel können im Falle der organischen Template organische Flüssigkeiten wie z.B. bei Polystyrol Tetrahydrofuran, oder saure oder basische wässrige Lösungen sein, wie z.B. bei Melaminformaldehydharzen HCl ^{[6]}. Insbesondere Silicapartikel lassen sich gut mit 1mol/l HF auflösen, da die entstehenden Produkte (SiF₆²⁻) leicht durch die Kapselmembran nach außen diffundieren, ohne die Kapselwand zu schädigen.^{[7]} 1 molare HF ist jedoch für viele Materialien nicht unproblematisch.

Daher ist insbesondere für empfindliche Wirkstoffe oder empfindliche Kapselwandmaterialien eine schonende Auflösung der Silicatemplate bei pH Werten von 3-6,5 bevorzugt. Dabei wird ein Fluoridsalz mit einer Konzentration von 1-5 mol/l mit einer Pufferlösung 1-5 mol/l auf einen gewünschten pH von 3 - 6 eingestellt. In dieser Reaktionsmischung lösen sich insbesondere poröse Silicatemplate bei genügender Reaktionszeit rückstandslos auf. Die Hexafluorosilikatanionen diffundieren problemlos auch durch dicke LbL Schichten aus den Kapseln heraus. Der bevorzugte pH-Wertbereich erstreckt sich von 3 oder 3,5 (untere Grenze) bis zu 6 oder 6,5.

Die beschriebene Methode zum Auflösen der Template kann unabhängig davon, ob es sich um poröse oder nichtporöse Mikropartikel handelt, verwendet werden und eignet sich insbesondere zum Auflösen von porösen und nicht porösen Silica- und Zeolithpartikeln. Diese Methode ist darüber hinaus grundsätzlich zum Auflösen derartiger Materialien geeignet, wobei diese Materialien im pH-Bereich von 3,5 bis 6 durch Fluoridsalze unter Anwesenheit von Pufferlösung, insbesondere eines Acetat/Essigsäurepuffers, aufgelöst werden. Diese Lösungsmethode eignet sich besonders für säureempfindliche Materialien, die entweder die Kapselwand bilden oder im Inneren eingeschlossen sind. Das betrifft viele Biopolymere wie z.B. Proteine, Enzyme, DNA, aber auch säureempfindliche Polymere oder Nanopartikel, wie z.B. Magnetit oder Quantum Dots (fluoreszierende Nanopartikel).

### Optionales Freisetzen des Wirkstoffs (Schritt E)

Nach dem Entfernen des Templates liegen mit einem Wirkstoff befüllte Mikrokapseln 12 (Figur 1A) oder mit einem inneren Gerüst 16 versehene Mikrokapseln 13 vor. Je nach der eingestellten Permeabilität der Kapselwand und der Größe der eingeschlossenen Wirkstoffe bleiben die Wirkstoffe permanent im Inneren der Mikrokapseln immobilisiert oder werden innerhalb eines definierten Zeitraumes freigesetzt.

### Beispiele

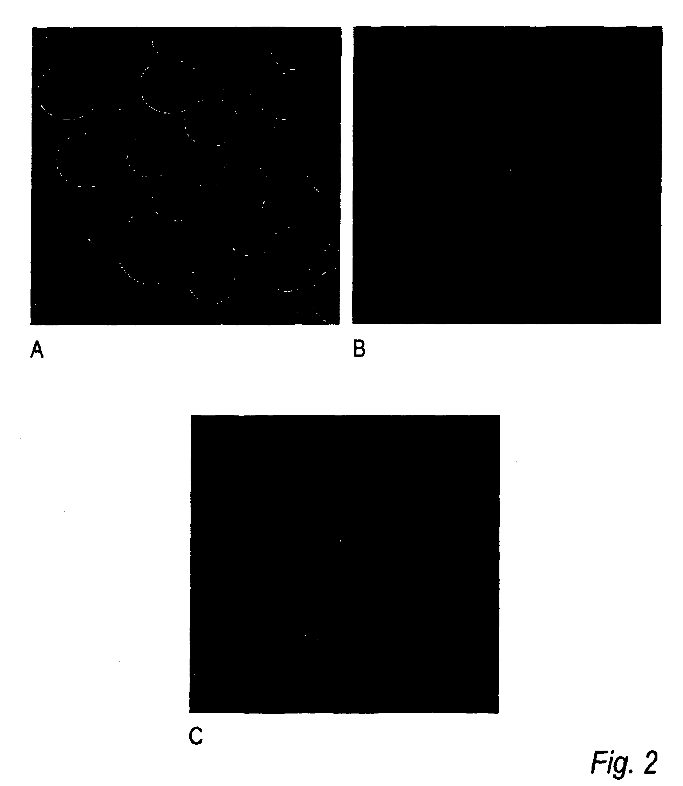

**1. Positiv geladenes Polymer:** 10 mg von sphärischen, porösen Silicatemplaten mit einem Durchmesser von 10 µm und einer Porengröße von 7 nm werden in 100 µL Wasser suspendiert (pH 6,5). Dazu werden 500 µL einer Lösung von 1 g/l Rhodamin gelabeltes Polyallylamin (PAH-Rho; PAH = Poly(allylamine hydrochloride)) mit einem Molekulargewicht von 70 000 g/mol gegeben und 12 h inkubiert. Der Überstand wird mit Pufferlösung weggewaschen. Sodann wird eine Lösung Fluorescein gelabeltes Chitosan (Chitosan-Flu) mit einem Molekulargewicht > 300 000 g/mol in 0,5 mol/l NaCl zu den Templaten gegeben und an der Oberfläche adsorbiert. Wie die konfokale Aufnahme zeigt, bildet es eine homogene Schicht an der Oberfläche und dringt kaum in die Template ein (Figur 2b). Nach der Grundierung werden alternierend 7 Schichten von PSS (Poly(sodium 4-styrenesulfonate)) und PAH mit Lösungen von 1 g/l Polymer in 0,5 mol/l Salz aufgebracht. Zwischen den Beschichtungsschritten wird 3 Mal mit Wasser gewaschen. Eine Analyse der erhaltenen CS-Partikel ergab im Inneren eine Konzentration von 7 g/l PAH-Rho (Figur 2a). Die Konzentrationen der Wirkstoffe im Inneren der CS-Partikel/Kapseln wurden mit der konfokalen Mikroskopie anhand von Vergleichslösungen über die Fluoreszenz bestimmt. Dabei kann die hohe Konzentration an Farbstoff im Inneren der Kapseln zu Self-Quenching führen, was geringere Konzentrationen vortäuscht.

Die CS-Partikel wurden mit 100 mL einer Lösung von 2 mol/l Natriumfluorid in 1 mol/l Acetatpuffer (pH 4) inkubiert. Nach 3 h haben sich die Template (Kerne) vollständig aufgelöst und die mit PAH gefüllten Kapseln bleiben zurück (Figur 2c). Nach mehreren Waschzyklen mit Wasser wurde im Inneren der Kapseln eine Konzentration von 6,3 g/l PAH-Rho bestimmt, die sich bei mehrwöchiger Lagerung nicht mehr veränderte.

Figur 2 zeigt hergestellte CS-Partikel und Kapseln mit positiv geladenem Polymer im Inneren; a) konfokales Bild von mit PAH-Rho befüllten CS-Partikeln, die mit Chitosan-Flu grundiert und mit 7 Schichten PAH/PSS beschichtet sind (Rhodaminkanal PMT2 600 V, Bildgröße 80 µm x 80 µm); b) konfokales Bild der CS-Partikel (Fluoresceinkanal PMT1 750 V, Bildgröße 80 µm x 80 µm); c) konfokales Bild von mit PAH-Rho befüllten Kapseln Chitosan(PSS/PAH)₃PSS nach Herauslösen des SiO₂ Templates (Rhodaminkanal PMT2 600V, Bildgröße 80 µm x 80 µm).

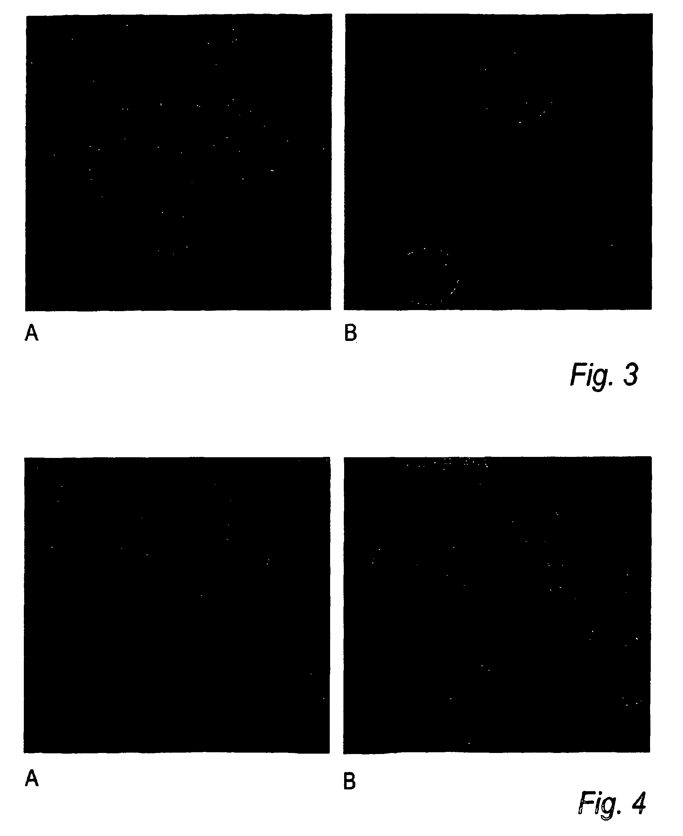

**2. Negativ geladenes Polymer:** 10 mg von sphärischen, porösen Silicatemplate mit einem Durchmesser von 10 µm und einer Porengröße von 7 nm werden in 100 µL Wasser suspendiert (pH 6,5). Anschließend werden die Template in einer 0,1 mol/l Lösung von FeCl₃ inkubiert. Nach drei Waschzyklen mit Wasser wurden 500 µL einer Lösung von 1 g/l Rhodamin gelabeltes Polystyrolsulfonat (PSS, MW 130 000 g/mol Capsulution Nanoscience AG) zugegeben und 12 h inkubiert. Das anionisch geladene PSS-Rho hat sich über das Fe³⁺ an der Oberfläche der Poren adsorbiert. Der PSS Überstand wird mit Wasser weggewaschen. Sodann wird eine Lösung von Chitosan-Flu mit einem Molekulargewicht > 300 000 g/mol in 0,5 mol/l NaCl zu den Templaten gegeben und an der Oberfläche adsorbiert. Nach der Grundierung werden alternierend 7 Schichten von PSS und PAH mit Lösungen von 1 g/l Polymer in 0,5 mol/l Salz aufgebracht. Zwischen den Beschichtungsschritten wird 3 Mal mit Wasser gewaschen. Im Inneren der so erhaltenen CS-Partikel wurde danach eine Konzentration von 2,3 g/l PSS-Rho bestimmt (Figur 3a). An der Wand ist die Konzentration an PSS besonders hoch, was auf eine verstärkte Adsorption an der inneren Oberfläche der Chitosangrundierung zurückzuführen ist. Die Silicatemplate werden mit 100 mL einer Lösung von 2 mol/l Natriumfluorid in 1 mol/l Acetatpuffer pH 4 herausgelöst. Nach 3 h haben sich die Template vollständig aufgelöst und die mit PSS gefüllten Kapseln bleiben zurück (Figur 3b). Im Inneren der Kapseln wurde eine Konzentration von 1,8 g/l PSS-Rho bestimmt, die bei mehrwöchiger Lagerung geringfügig abnahm.

Figur 3 zeigt hergestellte Kapseln mit negativ geladenem Polymer im Inneren; a) konfokales Bild von mit PSS-Rho befüllten CS-Partikeln, die Chitosan(PSS/PAH)₃PSS beschichtet sind (Rhodaminkanal PMT2 800 V, Bildgröße 80 µm x 80 µm); b) Konfokales Bild von mit PSS-Rho befüllten Kapseln aus Chitosan(PSS/PAH)₃PSS nach Herauslösen des SiO₂ Templates (Rhodaminkanal PMT2 850 V, Bildgröße 80 µm x 80 µm).

### 3. Zwitterionisches Protein Albumin

10 mg von sphärischen, porösen Silicatemplaten mit einem Durchmesser von 10 µm und einer Porengröße von 7 nm werden in 100 µL Wasser suspendiert (pH 6,5). Dazu werden 500 µL einer Lösung von 1 g/l Rhodamin gelabeltes Rinderserum-Albumin (TRITC-BSA, Sigma; BSA = Bovine Serum Albumin) in Acetat-Puffer (0,1 M, pH 5) gegeben und 12 h inkubiert. Der Überstand wird mit Pufferlösung weggewaschen, im Inneren der Template hat sich das Albumin deutlich akkumuliert. Sodann wird eine Lösung Chitosan-Flu mit einem Molekulargewicht > 300 000 g/mol in 0,5 mol/l NaCl zu den Templaten gegeben und an der Oberfläche adsorbiert. Nach der Grundierung werden alternierend 7 Schichten von PSS und PAH mit Lösungen von 1 g/l Polymer in 0,5 mol/l Salz aufgebracht. Zwischen den Beschichtungsschritten wird 3 Mal mit Wasser gewaschen. Im Inneren der erhaltenen CS-Partikel wurde eine Konzentration von 1,2 g/l BSA bestimmt (Figur 4a). Die Silicatemplate wurden in 100 mL einer Lösung von 2 mol/l Natriumfluorid in 1 mol/l Acetatpuffer bei einem pH von 5 herausgelöst. Nach 12 h haben sich die Template vollständig aufgelöst und die mit Albumin gefüllten Kapseln bleiben zurück (Figur 4b). Im Inneren der Kapseln wurde eine Konzentration von 1,4 g/l BSA bestimmt, die sich bei mehrwöchiger Lagerung nicht veränderte. Der höhere Wert in den Kapseln im Vergleich zu den CS-Partikeln resultiert entweder aus einer Abnahme des beim ersten Beispiel erwähnten SelfQuenchings oder aus einer Ablösung des BSA von der Wand ins Innere.

Figur 4 zeigt hergestellte Kapseln mit Protein im Inneren; a) konfokales Bild von mit BSA-Rho (BSA mit Rhodamin markiert) befüllten CS-Partikeln, die mit Chitosan(PSS/PAH)₃PSS beschichtet sind (Rhodaminkanal PMT 850V, Bildgröße 80 µm x 80 µm); b) Konfokales Bild von mit BSA-Rho befüllten Kapseln aus Chitosan(PSS/PAH)₃PSS nach dem Herauslösen des SiO₂ Templates (Rhodaminkanal PMT 900 V, Bildgröße 80 µm x 80 µm).

### 4. Sequentielle Einlagerung von 2 verschiedenen Wirkstoffen

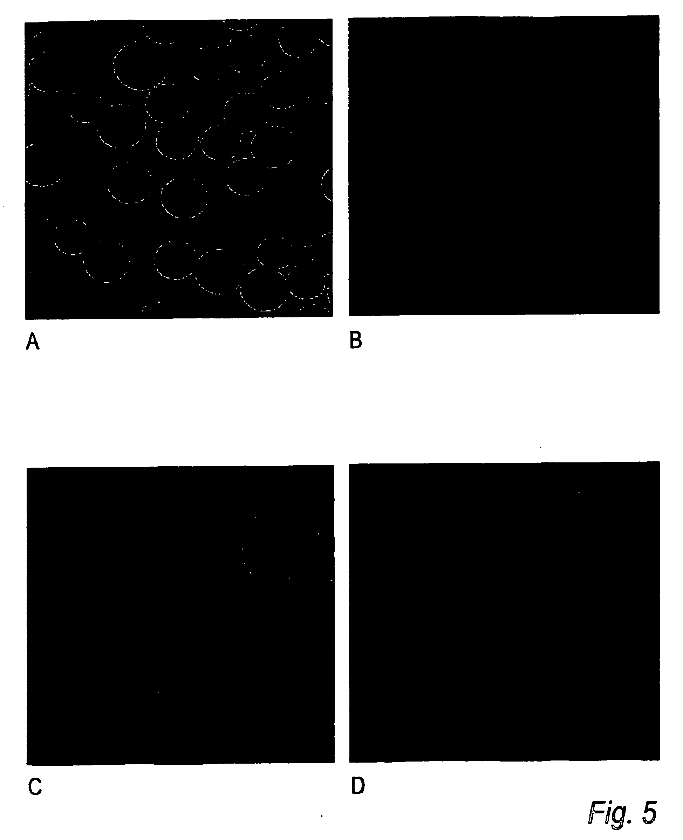

10 mg von sphärischen, porösen Silicatemplaten mit einem Durchmesser von 10 µm und einer Porengröße von 7 nm werden in 100 µL Wasser suspendiert (pH 6,5). Dazu werden 100 µL einer Lösung von 1 g/l mit Rhodamin gelabeltes Polyallylamin (PAH) mit einem Molekulargewicht von 70 000 g/mol gegeben und 12 h inkubiert. Das kationisch geladene PAH-Rho hat sich im Inneren der Partikel akkumuliert. Im Überstand befindet sich kein PAH mehr. Im nächsten Schritt wird eine Lösung von 500 µL Fluorescein gelabeltes Chitosan mit einem Molekulargewicht von 50 000 - 300 000 g/mol zu den Partikeln gegeben und weitere 12 h inkubiert. Nach dem Wegwaschen des Chitosanüberstandes wird eine Lösung von Chitosan mit einem Molekulargewicht > 300 000 g/mol in 0,5 mol/l NaCl zu den Partikeln gegeben und an der Oberfläche adsorbiert. Nach der Grundierung werden alternierend 7 Schichten von PSS und PAH mit Lösungen von 1 g/l Polymer in 0,5 mol/l Salz aufgebracht. Zwischen den Beschichtungsschritten wird 3 Mal mit Wasser gewaschen. Wie die konfokalen Aufnahmen zeigen (Figur 5a,b), befinden sich im Inneren der CS-Partikel 2,5 g/l PAH-Rho sowie 7 g/l niedermolekulares Chitosan-Flu. Die Silicatemplate wurden mit 100 mL einer Lösung von 2 mol/l Natriumfluorid in 1 mol/l Acetatpuffer pH 4 herausgelöst. Nach 3 h haben sich die Template vollständig aufgelöst und die mit PAH und Chitosan gefüllten Kapseln bleiben zurück (Figur 5c,d). Im Inneren der Kapseln wurden Konzentrationen von 1,7 g/l PAH-Rho und von 7 g/l Chitosan-Flu bestimmt, die sich bei mehrwöchiger Lagerung nicht veränderten.

Figur 5 zeigt konfokale Bilder von CS-Partikeln und Kapseln, die mit 2 positiv geladenen Polymeren PAH-Rho und niedermolekulares Chitosan-Flu gefüllt und mit Chitosan (PSS/PAH)₃PSS verkapselt sind; a) CS-Partikel im Rhodaminkanal PMT2 600 V, Bildgröße 80 µm x 80 µm; b) CS-Partikel im Fluoresceinkanal PMT1 500 V, Bildgröße 80 µm x 80 µm; c) Kapseln im Rhodaminkanal PMT2 700 V, Bildgröße 80 µm x 80 µm, d) Kapseln im Fluoresceinkanal PMT1 550 V, Bildgröße 80 µm x 80 µm.

### 5. Befüllung mit Nanopartikeln

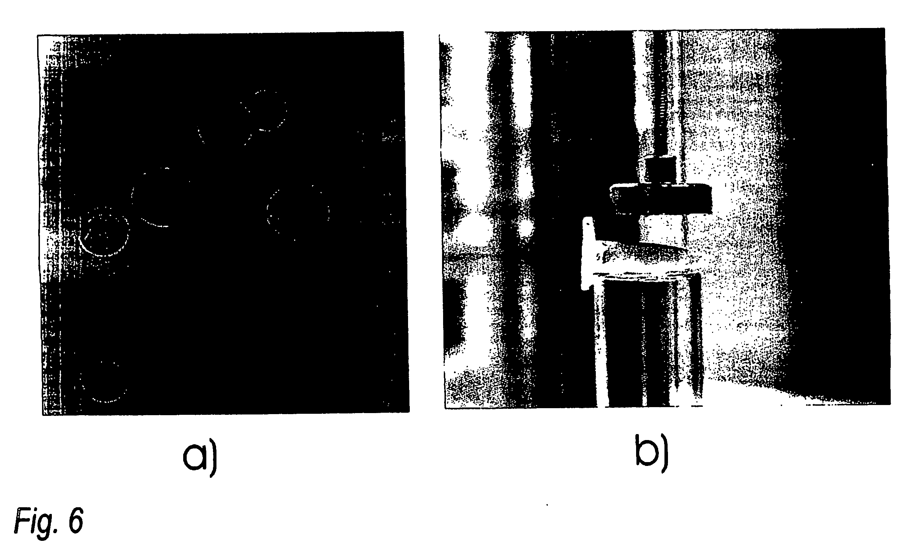

10 mg von sphärischen, porösen Silicatemplaten mit einem Durchmesser von 10 µm und einer Porengröße von 10 nm werden in 100 µL Wasser suspendiert (pH 6,5). Dazu werden 100 µL einer Lösung von 1 g/l positiv geladener Magnetit-Nanopartikel mit einem Durchmesser von 5-10 nm in einer Acetatpufferlösung pH 5,2 gegeben. Nach 12 h Inkubation wird der Überstand weggewaschen. Die porösen Template zeigen eine deutliche superparamagnetische Aktivität. Eine Lösung von Chitosan mit einem Molekulargewicht > 300 000 g/mol in 0,5 mol/l NaCl wird zu den Templaten gegeben und an der Oberfläche adsorbiert. Nach der Grundierung werden alternierend 7 Schichten von PSS und PAH mit Lösungen von 1 g/l Polymer in 0,5 mol/l Salz aufgebracht. Zwischen den Beschichtungsschritten wird 3 Mal mit Wasser gewaschen. Nach der Beschichtung hat sich die magnetische Aktivität nicht verändert. Die Silicatemplate wurden mit 100 mL einer Lösung von 2 mol/l Natriumfluorid in 1 mol/l Acetatpuffer pH 4,5 herausgelöst. Nach 12 h haben sich die Template vollständig aufgelöst und die mit Magnetit gefüllten Kapseln bleiben zurück (Figur 6a). Die magnetische Aktivität verringerte sich während der Auflösung kaum (Fig. 6b).

Figur 6 a zeigt konfokale Bilder von Kapseln, die mit positiv geladenen Magnetit-Nanopartikeln gefüllt und mit Chitosan(PSS/PAH)₃PSS verkapselt sind (80 µm x 80 µm). Figur 6b zeigt, wie die Kapseln in einem Eppendorf-Röhrchen mittels eines Magneten an der Oberseite gesammelt werden können.

### 6. Mikrokapseln mit einem festen Strukturgerüst im Inneren

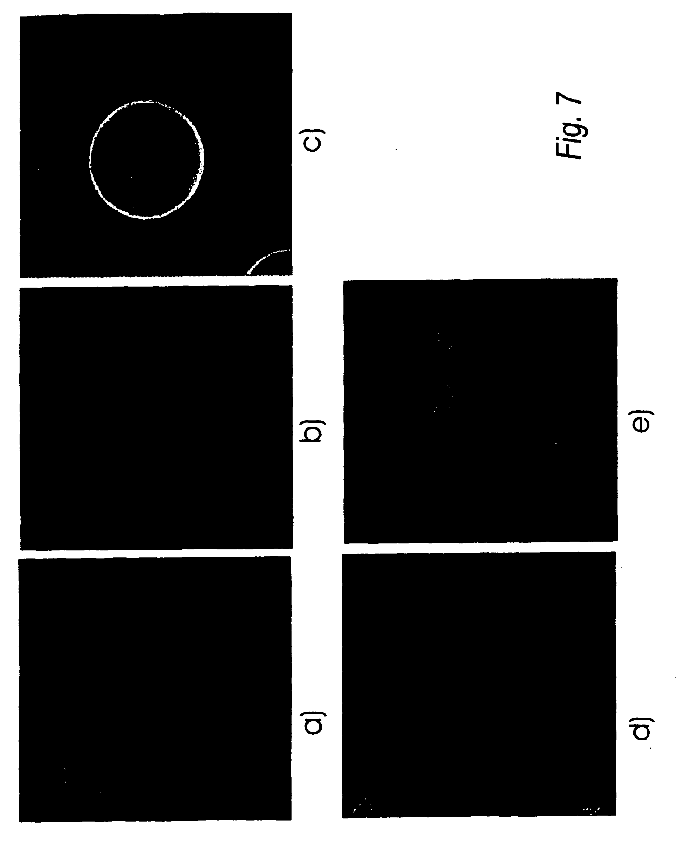

10 mg von sphärischen, porösen Silicatemplaten mit einem Durchmesser von 10 µm und einer Porengröße von 10 nm werden in 100 µL Wasser suspendiert (pH 6,5). Dazu werden 100 µL einer Lösung von 1 g/l mit Rhodamin gelabeltes Polyallylamin (PAH) mit einem Molekulargewicht von 15 000 g/mol in 0,5 mol/l NaCl gegeben und 60 min unter zeitweiliger Anwendung von Ultraschall inkubiert. Das kationisch geladene PAH-Rho hat sich im Inneren der Partikel akkumuliert. Der Überschuss an PAH-Rho wird weggewaschen. Danach wird mit PSS 20 000 g/mol in 0,5 mol/l Salz inkubiert und der Überstand weggewaschen. Dieses Verfahren wird 4 mal durchgeführt (8 Schichten). Danach wird eine Lösung von Chitosan mit einem Molekulargewicht > 300 000 g/mol in 0,5 mol/l NaCl zu den Partikeln gegeben und an der Oberfläche adsorbiert. Nach der Grundierung werden alternierend 7 Schichten von PSS und PAH 70 000 (mit dem Farbstoff Cy5 gelabelt) mit Lösungen von 1 g/l Polymer in 0,5 mol/l Salz aufgebracht. Zwischen den Beschichtungsschritten wird mit Wasser gewaschen. Wie die konfokalen Aufnahmen zeigen (Figur 7a,b) befindet sich im Inneren der CS-Partikel PAH-Rho, während das Cy5 gelabelte PAH die Hülle bildet Als Konzentration wurde 21,5 g/l PAH-Rho bestimmt. Die Silicatemplate wurden mit 100 mL einer Lösung von 2 mol/l Natriumfluorid in 1 mol/l Acetatpuffer pH 4 herausgelöst. Nach 3 h haben sich die Template vollständig aufgelöst und die mit dem PSS/PAH-Rho gefüllten Kapseln bleiben zurück (Figur 7c). Im Inneren der Kapseln wurden Konzentrationen von 16,9 g/l PAH-Rho bestimmt, die wegen des in dem Komplexgerüst auftretenden Selfquenchings garantiert deutlich höher sind. Im Gegensatz zu den herkömmlichen Kapseln kollabieren diese Kapseln durch das stabile Gerüst beim Trocknen nicht (Figur 7d,e). Weiterhin wurde die Ortsstabilität des filigranen Gerüstes im Inneren durch das Bleichen von Bereichen (2 Punkte links und rechts vom Zentrum) und nachfolgendes Scannen mit dem konfokalen Mikroskop nachgewiesen (Figur 7 c).

Figur 7 zeigt konfokale Bilder von CS-Partikeln und Kapseln die mit einem PAH-Rho/PSS Komplex gefüllt und mit Chitosan(PSS/PAH)₃PSS umhüllt sind a) CS-Partikel im Rhodaminkanal PMT2 600 V, Bildgröße 40 µm x 40 µm; b) CS-Partikel im Cy5 Kanal PMT1 500 V, Bildgröße 40 µm x 40 µm; c) Kapsel im Rhodamin- und Cy5 Kanal überlagert, in die Kapsel wurden mit hoher Laserleistung 2 Löcher gebrannt, die orts-stabil sind, Bildgröße 40 µm x 40 µm, d) Kapseln nach Beispiel 1 hergestellt (PAH-Rho befüllt) nach dem Trocknen, Bildgröße 40 µm x 40 µm e) Kapseln mit PAH-Rho/PSS Gerüst nach dem Trocknen, Bildgröße 40 µm x 40 µm

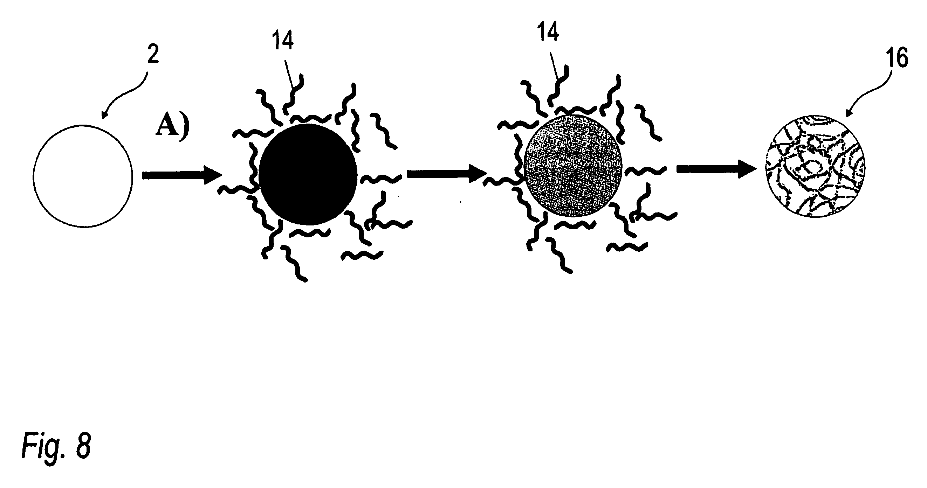

Figur 8 zeigt einzelne Verfahrensschritte zur Herstellung von Mikrotemplaten 16, die hier aus einem filigranen Gerüst von Polyelektrolyt- und/oder Nanopartikelschichten 14 bestehen. Dazu werden poröse Template 2 mit alternierend geladenen Polyelektrolyt- und/oder Nanopartikelschichten 14 befüllt, d.h. diese Materialien beschichten die innere Oberfläche (Porenoberfläche) der Template 2 und ggf. auch die äußere Oberfläche der Template. Nach dem Auflösen der Template 2 bleiben Mikrotemplate 16 zurück, die von teilweise bzw. weitgehend geschlossenen Polyelektrolyt- und/oder Nanopartikelschichten umgeben sein können.

### Bezugszeichenliste

- 2: poröses Templat
- 4: Wirkstoff
- 5: mit Wirkstoff befülltes Templat
- 6: Grundierungsschicht
- 8: Schichten der Kapselhülle
- 9: Kapselhülle
- 10: CS-Partikel
- 12, 13: Mikrokapsel
- 14: Polyelektrolyte / Nanopartikel
- 16: inneres Gerüst / Mikrotemplat

### Literatur

[1] E. Donath, G. B. Sukhorukov, F. Caruso, S. A. Davis, H. Möhwald, Angewandte Chemie-International Edition 1998, 37, 2202-2205.
[2] L. Dähne, S. Leporatti, E. Donath, H. Möhwald, Journal of the American Chemical Society 2001, 123, 5431-5436.
[3] A. A. Antipov, G. B. Sukhorukov, S. Leporatti, I. L. Radtchenko, E. Donath, H. Möhwald, Colloids and Surfaces a-Physicochemical and Engineering Aspects 2002, 198, 535-541.
[4] G. Ibarz, L. Dähne, E. Donath, H. Möhwald, Advanced Materials 2001, 13, 1324-1327.
[5] I. L. Radtchenko, G. B. Sukhorukov, H. Möhwald, Colloids and Surfaces a-Physicochemical and Engineering Aspects 2002, 202, 127-133.
[6] H. Möhwald, E. Donath, G. Sukhorukov in MultilayerThin Films (Ed.: J. B. Schlenoff), Wiley VCH, New York / Basel, 2003, pp. 363-391.
[7] L. Dähne, C. Peyratout in Encyclopaedia of Nanoscience and Nanotechnology Marcel Dekker, Inc., New York 2004
[8] D. G. Shchukin, G.B. Sukhorukov, H. Möhwald Angewandte Chemie International Edition 2003, 42, 4472-4475.
[9] D.V. Volodkin, A. I. Petrov, M. Prevot, G. B. Sukhorukov, Langmuir 2004, 20, 3398-3406.

## Patentansprüche

1. Verfahren zur Herstellung von CS-Partikeln und/oder Mikrokapseln mit den Schritten:
- poröse Template werden bereitgestellt, wobei es sich bei den Templaten um poröse organische und/oder anorganische Mikropartikel mit einem Durchmesser kleiner als 100 µm handelt
- zumindest ein zu verkapselnder Wirkstoff wird in den porösen Templaten adsorbiert
- zumindest eine Grundierungsschicht wird auf die porösen Template aufgebracht und
- eine Kapselhülle wird um die mit der Grundierungsschicht versehenen porösen Template durch Aufbringen von alternierend geladenen Polyelektrolyt- und/oder Nanopartikelschichten auf die porösen Template gebildet,
- wobei die Grundierungsschicht aus einem Material gebildet wird, das die Poren der porösen Template verschließt und für die zur Herstellung der Kapselhülle verwendeten Beschichtungsmaterialien weitgehend undurchlässig ist.

2. Verfahren nach Anspruch 1, **dadurch gekennzeichnet, dass** die porösen Template Poren mit einer Porenweite von 0,3 nm - 100 nm und bevorzugt von 1 nm - 30 nm aufweisen.

3. Verfahren nach einem der vorhergehenden Ansprüche, **dadurch gekennzeichnet, dass** es sich bei den Templaten um poröse Silicapartikel und/oder um poröse Zeolithpartikel und/oder um poröse Polystyrolpartikel handelt.

4. Verfahren nach Anspruch 3, **dadurch gekennzeichnet, dass** die porösen Silicapartikel im Größenbereich von 100 nm bis 100 µm und bevorzugt von 500 nm bis 30 µm liegen.

5. Verfahren nach Anspruch 3, **dadurch gekennzeichnet, dass** die porösen Zeolithpartikel eine Porenweite von 0,3 nm bis 10 nm aufweisen.

6. Verfahren nach einem der vorhergehenden Ansprüche, **dadurch gekennzeichnet, dass** es sich bei dem zumindest einen zu verkapselnden Wirkstoff um zumindest ein Polymer und/oder Protein und/oder organisches Molekül mit einem Molekulargewicht über 100 g/mol und/oder Nanopartikel und insbesondere um ein Enzym und/oder Katalysator und/oder Farbstoff und/oder pharmazeutischen bzw. kosmetischen Wirkstoff handelt.

7. Verfahren nach einem der vorhergehenden Ansprüche, **dadurch gekennzeichnet, dass** zumindest ein Hilfsstoff zur Vermittlung der Adsorption des zumindest einen Wirkstoffes verwendet wird.

8. Verfahren nach einem der vorhergehenden Ansprüche, **dadurch gekennzeichnet, dass** als Wirkstoff Polyelektrolyte und/oder Nanopartikel verwendet werden und dass die Oberfläche der Porenhohlräume durch mehrere Schichten von alternierend geladenen Polyelektrolyten und/oder Nanopartikeln beschichtet wird.

9. Verfahren nach einem der vorhergehenden Ansprüche, **dadurch gekennzeichnet, dass** die porösen Template in einer Lösung bereitgestellt werden und zusätzlich oder alternativ zum Hilfsstoff durch Änderung des pH-Werts der Lösung die Adsorption des zumindest einen Wirkstoffes gesteuert wird.

10. Verfahren nach einem der vorhergehenden Ansprüche, **dadurch gekennzeichnet, dass** die porösen Template nach Bildung der Kapselhülle aufgelöst werden und dadurch die Mikrokapseln entstehen.

11. Verfahren nach Anspruch 10, **dadurch gekennzeichnet, dass** die Silica- und/oder Zeolithtemplate durch Fluoridsalze in Gegenwart einer Pufferlösung mit einem pH-Wert zwischen 3,5 und 6 aufgelöst werden.

12. CS-Partikel mit
- einem Durchmesser kleiner als 100 µm
- einem porösen Kern in dem zumindest ein Wirkstoff adsorbiert ist
- einer Grundierungsschicht, die den porösen Kern umgibt und
- einer Kapselhülle aus mehreren Schichten alternierend geladener Polyelektrolyt- und/oder Nanopartikelschichten,
- wobei die Grundierungsschicht aus einem Material besteht, das die Poren des porösen Kerns verschließt und für die Beschichtungsmaterialen, aus denen die Kapselhülle besteht, weitgehend undurchlässig ist.

13. CS-Partikel nach Anspruch 12, **dadurch gekennzeichnet, dass** die porösen Kerne Poren mit einer Porenweite von 0,3 nm - 100 nm und bevorzugt von 1 nm - 30 nm aufweisen.

14. CS-Partikel nach Anspruch 12 oder 13, **dadurch gekennzeichnet, dass** es sich bei den Kernen um poröse organische und/oder anorganische Mikropartikel mit einem Durchmesser kleiner als 100 µm handelt.

15. CS-Partikel nach einem der Ansprüche 12 bis 14, **dadurch gekennzeichnet, dass** es sich bei den Kernen um poröse Silicapartikel und/oder um poröse Zeolithpartikel und/oder um poröse Polystyrolpartikel handelt.

16. CS-Partikel nach einem der Ansprüche 12 bis 15, **dadurch gekennzeichnet, dass** es sich bei den Kernen um poröse Silicapartikel im Größenbereich von 100 nm bis 100 µm und bevorzugt von 500 nm bis 30 µm handelt.

17. CS-Partikel nach einem der Ansprüche 12 bis 16, **dadurch gekennzeichnet, dass** es sich bei den Kernen um poröse Zeolithpartikel mit einer Porenweite von 0,3 nm bis 10 nm handelt.

18. Mikrokapseln mit
- einem Durchmesser kleiner als 100 µm
- einer Kapselhülle aus mehreren Schichten alternierend geladener
- Polyelektrolyt- und/oder Nanopartikelschichten;
- einer Grundierungsschicht an der Innenseite der Kapselhülle und
- einem inneren Gerüst aus Polyelektrolytkomplexen und/oder Polyelektrolyt/Nanopartikelkomplexen, das von der Grundierungsschicht und der Kapselhülle umgeben ist.

19. CS-Partikel oder Mikrokapseln nach einem der Ansprüche 12 bis 18, **dadurch gekennzeichnet, dass** die Grundierungsschicht und die Kapselhülle aus unterschiedlichen Materialien bestehen

20. CS-Partikel oder Mikrokapseln nach einem der Ansprüche 12 bis 19, **dadurch gekennzeichnet, dass** es sich bei dem zumindest einen Wirkstoff um Proteine und/oder Polymere und/oder Enzyme und/oder Katalysatoren und/oder Farbstoffe und/oder Nanopartikel handelt.

21. Verfahren zur Herstellung von Mikrokapseln mit den Schritten:
- zumindest ein poröses Templat wird bereitgestellt, wobei es sich bei dem Templat um ein poröses organisches und/oder anorganisches Mikropartikel mit einem Durchmesser kleiner als 100 µm handelt
- die Oberfläche der Porenhohlräume des porösen Templates wird mit mehreren Schichten von alternierend geladenen Polyelektrolyten und/oder Nanopartikeln beschichtet
- zumindest eine Grundierungsschicht wird auf das poröse Templat aufgebracht;
- eine Kapselhülle wird um das mit der Grundierungsschicht versehene poröse Templat durch Aufbringen von alternierend geladenen Polyelektrolyt- und/oder Nanopartikelschichten auf das poröse Templat gebildet, wobei die Grundierungsschicht aus einem Material gebildet wird, das die Poren des porösen Templats verschließt und für die zur Herstellung der Kapselhülle verwendeten Beschichtungsmaterialien weitgehend undurchlässig ist und
- das poröse Templat wird aufgelöst.

22. Verwendung der CS-Partikel und/oder Mikrokapseln nach einem der Ansprüche 12 bis 20 und/oder Verwendung der nach einem der Ansprüche 1 bis 11 und 21 hergestellten CS-Partikel und/oder Mikrokapseln
- zur Verkapselung von Stoffen in den Bereichen der Diagnostik, Sensorik und/oder
- zur selektiven Akkumulation von Stoffen aus Lösungen für Anwendungen in der Wasserreinigung, Diagnostik, Nuklearchemie etc. und/oder
- zum Einschluss katalytisch wirkender Stoffe insbesondere Metalle und/oder Metalloxide und/oder Enzyme zur Katalyse chemischer und biochemischer Reaktionen und/oder
- zur Verkapselung von Nanopartikeln, insbesondere zur Herstellung fluoreszenter oder magnetischer Mikrokapseln, für diagnostische oder medizinische Anwendungen und/oder
- zur Verkapselung und Freisetzung von Wirkstoffen in der pharmazeutischen und kosmetischen Industrie und/oder
- zu Separationszwecken, z.B. in der Chromatographie und/oder
- zu Anwendungen in der Nahrungsmittelindustrie und der Land- und Forstwirtschaft.

## Claims

1. A method for manufacturing of CS particles and/or microcapsules with the steps of:
- porous templates are provided, wherein the templates are porous organic and/or inorganic microparticles with a diameter smaller than 100 µm
- at least one active agent to be encapsulated is adsorbed in the porous templates
- at least one layer of grounding is applied onto the porous templates and
- a capsule shell is generated around the porous templates furnished with the layer of grounding by applying layers of alternatingly charged polyelectrolytes and/or nanoparticles onto the porous templates,
- whereby the layer of grounding is generated from a material which closes the pores of the porous templates and is essentially impermeable for the coating materials used for the manufacturing of the capsule shell.

2. The method according to claim 1, **characterized in that** the pores of the porous templates have a pore width from 0.3 nm to 100 nm and preferably from 1 nm to 30 nm.

3. The method according to any one of the preceding claims **characterized in that** the templates are porous silica particles and/or porous zeolite particles and/or porous polystyrene particles.

4. The method according to claim 3, **characterized in that** the porous silica particles are in a size range from 100 nm to 100 µm and preferably from 500 nm to 30 µm.

5. The method according to claim 3 **characterized in that** the porous zeolite particles have a pore width from 0.3 nm to 10 nm.

6. The method according to any one of the preceding claims, **characterized in that** the at least one active agent to be encapsulated is at least one polymer and/or protein and/or organic molecule with a molecular weight above 100 g/mol and/or nanoparticle and particularly an enzyme and/or catalyst and/or dye and/or pharmaceutical or cosmetic agent.

7. The method according to any one of the preceding claims, **characterized in that** at least one additive is used for mediating the adsorption of the at least one active agent.

8. The method according to any one of the preceding claims, **characterized in that** polyelectrolytes and/or nanoparticles are used as the active agent and that the surface of the pore volume is covered with several layers of alternatingly charged polyelectrolytes and/or nanoparticles.

9. The method according to any one of the preceding claims, **characterized in that** the porous templates are provided in a solution and additionally or alternatively to the additive the adsorption of the at least one active agent is controlled by changing the pH of the solution.

10. The method according to any one of the preceding claims, **characterized in that** the porous templates are dissolved after the generation of the capsule shell and thereby the microcapsules are generated.

11. The method according to claim 10, **characterized in that** the silica and/or zeolite templates are dissolved by fluoride salts in the presence of a buffer solution with a pH between 3.5 and 6.

12. CS particles with
- a diameter smaller than 100 µm
- a porous core in which at least one active agent is adsorbed
- a layer of grounding enclosing the porous core and
- a capsule shell of several layers of alternatingly charged polyelectrolyte and/or nanoparticle layers,
- wherein the layer of grounding consists of a material which closes the pores of the porous core and is essentially impermeable for the coating materials which the capsule shell consists of.

13. CS particles according to claim 12, **characterized in that** the pores of the porous cores have a pore width from 0.3 nm to 100 nm and preferably from 1 nm to 30 nm.

14. CS particles according to claim 12 or 13, **characterized in that** the cores are porous organic and/or inorganic microparticles with a diameter smaller than 100 µm.

15. CS particles according to any one of claims 12 or 14, **characterized in that** the cores are porous silica particles and/or porous zeolite particles and/or porous polystyrene particles.

16. CS particles according to any one of claims 12 to 15, **characterized in that** the cores are porous silica particles in the size range from 100 nm to 100 µm and preferably from 500 nm to 30 µm.

17. CS particles according to any one of claims 12 to 16, **characterized in that** the cores are porous zeolite particles with a pore width from 0.3 nm to 10 nm.

18. Microcapsules with
- a diameter smaller than 100 µm
- a capsule shell of several layers of alternatingly charged polyelectrolyte and/or nanoparticle layers;
- a layer of grounding at the inner surface of the capsule shell and
- an inner scaffold of polyelectrolyte complexes and/or polyelectrolyte/nanoparticle complexes which is enclosed by the layer of grounding and the capsule shell.

19. CS particles or microcapsules according to any one of claims 12 to 18, **characterized in that** the layer of grounding and the capsule shell consist of different materials.

20. CS particles or microcapsules according to any one of claims 12 to 19, **characterized in that** the at least one active agent is proteins and/or polymers and/or enzymes and/or catalysts and/or dyes and/or nanoparticles.

21. A method for manufacturing microcapsules with the steps of:
- at least one porous template is provided, wherein the template is a porous organic and/or inorganic microparticle with a diameter smaller than 100 µm
- the surface of the pore volume of the porous templates is coated with several layers of alternatingly charged polyelectrolytes and/or nanoparticles
- at least one layer of grounding is applied onto the porous template;
- a capsule shell is generated around the porous template furnished with the layer of grounding by applying layers of alternatingly charged polyelectrolytes and/or nanoparticles onto the porous template, wherein the layer of grounding is of a material which closes the pores of the porous template and is essentially impermeable for the coating materials used for the manufacturing of the capsule shell and
- the porous template is dissolved.

22. Use of CS particles and/or microcapsules according to any one of claims 12 to 20 and/or use of CS particles and/or microcapsules manufactured according to any one of claims 1 to 11 and 21
- for encapsulating substances in the field of diagnostics, sensor technology and/or
- for selectively accumulating substances from solutions for applications in water purification, diagnostics, nuclear chemistry etc. and/or
- for encapsulating catalytically active substances, particularly metals and/or metal oxides and/or enzymes for the catalysis of chemical and biochemical reactions and/or
- for encapsulating nanoparticles, particularly for the manufacturing of fluorescent or magnetic microcapsules for diagnostic or medical applications and/or
- for encapsulating and release of active agents in the pharmaceutical and cosmetic industry and/or
- for separation purposes, e.g. in chromatography and/or
- for applications in the food industry and agriculture and forestry.

## Revendications

1. Procédé de production de particules de CS et/ou de microcapsules comprenant les étapes suivantes :
- des matrices poreuses sont produites, les matrices étant des microparticules organiques et/ou anorganiques poreuses présentant un diamètre inférieur à 100 µm,
- au moins une substance active à encapsuler est adsorbée dans les matrices poreuses,
- au moins une couche d'apprêt est appliquée sur les matrices poreuses et
- une enveloppe de capsule est formée autour des matrices poreuses dotée de la couche d'apprêt par application de couches chargées de façon alternative de polyélectrolytes et/ou de nanoparticules sur les matrices poreuses,
- la couche d'apprêt étant formée d'un matériau qui ferme les pores des matrices poreuses et étant largement imperméable aux matériaux de revêtement utilisés pour la production de l'enveloppe de capsule.

2. Procédé selon la revendication 1, **caractérisé en ce que** les matrices poreuses présentent des pores ayant une largeur de pore de 0,3 nm à 100 nm et de préférence, de 1 nm à 30 nm.

3. Procédé selon l'une des revendications précédentes, **caractérisé en ce que** les matrices sont des particules de silice poreuses et/ou des particules de zéolithe poreuses et/ou des particules de polystyrène poreuses.

4. Procédé selon la revendication 3, **caractérisé en ce que** les particules de silice poreuses se situent dans un ordre de grandeur de 100 nm à 100 µm et de préférence de 500 nm à 30 µm.

5. Procédé selon la revendication 3, **caractérisé en ce que** les particules de zéolithe poreuses présentent une largeur de pore de 0,3 nm à 10 nm.

6. Procédé selon l'une des revendications précédentes, **caractérisé en ce qu'**au moins la substance active à encapsuler est au moins un polymère et/ou une protéine et/ou une molécule organique présentant un poids moléculaire supérieur à 100 g/mole et/ou des nanoparticules et en particulier une enzyme et/ou un catalyseur et/ou un colorant et/ou une substance active pharmaceutique ou cosmétique.

7. Procédé selon l'une des revendications précédentes, **caractérisé en ce que** l'on utilise au moins un adjuvant pour assurer l'adsorption d'au moins la substance active.

8. Procédé selon l'une des revendications précédentes, **caractérisé en ce que** l'on utilise comme substance active, des polyélectrolytes et/ou des nanoparticules et **en ce que** la surface de l'espace creux des pores est revêtue par plusieurs couches de polyélectrolytes et/ou de nanoparticules chargées de façon alternative.

9. Procédé selon l'une des revendications précédentes, **caractérisé en ce que** les matrices poreuses sont proposées dans une solution et en outre ou en variante de l'adjuvant, l'adsorption d'au moins une substance active est entraînée par la modification du pH de la solution.

10. Procédé selon l'une des revendications précédentes, **caractérisé en ce que** les matrices poreuses sont dissoutes après la formation de l'enveloppe de capsule et ainsi sont formées les microcapsules.

11. Procédé selon la revendication 10, **caractérisé en ce que** les matrices de silice et/ou de zéolithe sont dissoutes par des sels de fluorure en présence d'une solution tampon à un pH entre 3,5 et 6.

12. Particules de CS comportant
- un diamètre inférieur à 100 µm
- un noyau poreux qui est adsorbé dans au moins une substance active
- une couche d'apprêt qui entoure le noyau poreux et
- une enveloppe de capsule de plusieurs couches constituées de couches de polyélectrolytes et/ou de nanoparticules chargées de façon alternative
- la couche d'apprêt consistant en un matériau qui ferme les pores du noyau poreux et étant imperméable aux matériaux de revêtement qui constituent l'enveloppe de capsule.

13. Particules de CS selon la revendication 12, **caractérisées en ce que** les noyaux poreux présentent une largeur de pore de 0,3 nm à 100 nm et de préférence de 1 nm à 30 nm.

14. Particules de CS selon la revendication 12 ou 13, **caractérisées en ce que** les noyaux sont des microparticules organiques et/ou anorganiques poreuses présentent un diamètre inférieur à 100 µm.

15. Particules de CS selon l'une des revendications 12 à 14, **caractérisées en ce que** les noyaux sont des particules de silice poreuses et/ou des particules de zéolithe poreuses et/ou des particules de polystyrène poreuses.

16. Particules de CS selon l'une des revendications 12 à 15, **caractérisées en ce que** les noyaux sont des particules de silice poreuses dans un ordre de grandeur de 100 nm à 100 µm et de préférence de 500 nm à 30 µm.

17. Particules de CS selon l'une des revendications 12 à 16, **caractérisées en ce que** les noyaux sont des particules de zéolithe poreuses présentant une largeur de pore de 0,3 nm à 10 nm.

18. Microcapsules présentant
- un diamètre inférieur à 100 µm
- une enveloppe de capsule de plusieurs couches constituées de couches de polyélectrolytes et/ou de nanoparticules chargées de façon alternative
- la couche d'apprêt sur la face interne de l'enveloppe de capsule et
- un squelette interne constitué de complexes de polyélectrolytes et/ou de complexes de polyélectrolytes/nanoparticules, qui est entouré par la couche d'apprêt et l'enveloppe de capsule.

19. Particules de CS ou microcapsules selon l'une des revendications 12 à 18, **caractérisées en ce que** la couche d'apprêt et l'enveloppe de capsules consistent en matériaux différents.

20. Particules de CS ou microparticules selon l'une des revendications 12 à 19, **caractérisées en ce qu'**au moins la substance active consiste en protéines et/ou polymères et/ou enzymes et/ou catalyseurs et/ou colorants et/ou nanoparticules.

21. Procédé de production de microcapsules comportant les étapes suivantes :
- au moins une matrice poreuse est produite, la matrice étant une microparticule organique et/ou anorganique poreuse présentant un diamètre inférieur à 100 µm,
- la surface de l'espace creux des pores de la matrice poreuse est revêtue avec plusieurs couches chargées de façon alternative de polyélectrolytes et/ou de nanoparticules,
- au moins une couche d'apprêt est appliquée sur la matrice poreuse ;
- une enveloppe de capsule est formée autour de la matrice poreuse dotée de la couche d'apprêt par application de couches chargées de façon alternative de polyélectrolytes et/ou de nanoparticules sur la matrice poreuse, la couche d'apprêt étant formée d'un matériau qui ferme les pores de la matrice poreuse et étant largement imperméable aux matériaux de revêtement utilisés pour la production de l'enveloppe de capsule,
- la matrice poreuse est dissoute.

22. Utilisation de particules de CS et/ou de microparticules selon l'une des revendications 12 à 20 et/ou utilisation des particules de CS et/ou microparticules produites selon l'une des revendications 1 à 11 et 21
- pour l'encapsulation de substances dans les domaines du diagnostic, de l'analyse sensorielle et/ou
- pour l'accumulation sélective de substances issues de solutions pour les utilisations dans l'épuration de l'eau, le diagnostic, la chimie nucléaire etc. et/ou
- pour l'inclusion de substances agissant au niveau catalytique, en particulier des métaux et/ou des oxydes métalliques et/ou des enzymes pour la catalyse de réactifs chimiques et biochimiques et/ou
- pour l'encapsulation de nanoparticules, en particulier pour la production de microcapsules fluorescentes ou magnétiques, pour les utilisations diagnostiques et médicales et/ou
- pour l'encapsulation et la libération de substances actives dans l'industrie pharmaceutique et cosmétique et/ou
- à des fins de séparation, par exemple dans la chromatographie et/ou
- pour les utilisations dans l'industrie agro-alimentaires et l'agriculture et la sylviculture.
